# EUROPEAN PATENT APPLICATION

(11) **EP 1 790 618 A1**
(43) Date of publication of application: **30.05.2007**
(21) Application number: 05111341.3
(22) Date of filing: 25.11.2005
(51) Int. Cl.: C02F 1/42, C02F 1/78, C01B 13/11, A61L 2/20

(54) **Apparatus for deionizing drinkable water.**

(71) Applicant: Osmotek Srl, 20149 Milano (IT)
(72) Inventor: Barbera, Ivano, 13875, Ponderano (Bi) (IT); Barbera, Paolo, 20095, Cusano Milanino (Mi) (IT); Delsante, Giovanna, 20040, Busnago (IT); Valverde, Massimo, 20124, Milano (IT)
(74) Representative: Serravalle, Marco

(57) **Abstract**

The invention concerns an apparatus for sweetening drinkable water for domestic use, comprising a vessel containing the ion exchange resins, a vessel containing the reservoir of salt for the regeneration of the resins, a valve for managing the regeneration phases and an ozone generator which is able to provide a flux of air or water containing from 0.010 to 1.0 mg/l of ozone. Another aspect of the invention concerns an ozone generator for use in an apparatus for the treatment of domestic water comprising a double glass tube internally and externally coated with electrodes, to whom a tension of about 13-15 kV is applied, and a pump which generates a flux through the glass tube, characterized in that the amount of ozone produced is comprised between 0.1 g/h and 5 g/h.

## Description

The present invention concerns a domestic apparatus for sweetening drinkable water by the use of ion exchange resins. More particularly, the invention concerns a regeneration and disinfection system for the resins of the above mentioned apparatus, which system allows to keep the apparatus sterile by killing the possible bacterial flora formed inside the apparatus during the working cycle.

Apparata for sweetening water based on ion exchange resins are well known in the art. Such apparata consist normally of a vessel wherein the ion exchange resins are located and which, under normal working conditions, is directly connected to the water net. Periodically, the resins require a regeneration cycle. For such purpose, they are excluded from the water line and undergo a series of washings. The problem of bacterial flora formation, and thus the need of its killing, it is well known and was taken into account in the European regulations, which require that said apparata undergo every 4 days as a maximum a regeneration and disinfection cycle. Disinfection is usually performed by chlorine generation by means of a pair of electrodes directly immersed in the brine used for the regeneration of the resins. However, the results obtained by these methods are not satisfactory. In fact, the ion exchange resins generally used are based on a copolymer styrene-divinylbenzene. These copolymers are very sensitive to the presence of chlorine and, at room temperature, are already damaged by the presence of 0.1 mg/l of chlorine. It is therefore evident that the use of chlorine generators either does not produce a meaningful amount of chlorine, in this way allowing a long life to the resins but producing an irrelevant disinfecting action, or it generates a chlorine amount of the order of a few mg/l, in this way degrading the resin in a short time.

It has been surprisingly found that the introduction of very small amounts of ozone not only allows an effective disinfection of the apparata, but it improves the working conditions of the resins allowing them a long life.

The disinfection of drinkable water by using ozone, is a well known system but, until now, exclusively limited to large structures. Ozone has not been considered up till now for domestic uses probably because of the problems connected to its use. In fact, it is well known that ozone is a toxic substance whose presence in the air at concentration of 0.3 mg/l results in airway inflammation. Existing regulations limit the concentration of ozone in working or domestic environments to values lower than 0.05-0.1 mg/l. Consequently, the use of apparata generating ozone in a domestic environment, was considered with extreme caution because of the risk that the presence of an ozone generator in a domestic environment could lead to ozone concentration higher than the admissible limit.

This kind of caution is also justified by the fact that industrially used apparata normally generate ozone at a concentration of several mg/l, thus much above the toxicity limits.

The present invention has been made possible thanks to the use in the apparatus itself of a new ozone generator which, besides being of very limited dimension, generates ozone at concentration of the order of 0.020-0.040 mg/l, which values are much lower than the toxicity limits of ozone.

An embodiment of the invention concerns an ozone generator for use in an apparatus for the treatment of domestic water comprising a double glass tube internally and externally coated with electrodes, to whom a tension of about 13-15 kV is applied, an electric circuit to feed the electrodes with a tension of about 13-15 kV, and a pump which generates an air flux through the glass tube. The ozone concentration at the exit of the ozone generator is in the range of 0.10 to 1.0 mg/l depending on the working condition of the ozone generator, and amount of ozone produced is comprised between 0.10 g/h and 5.0 g/h.

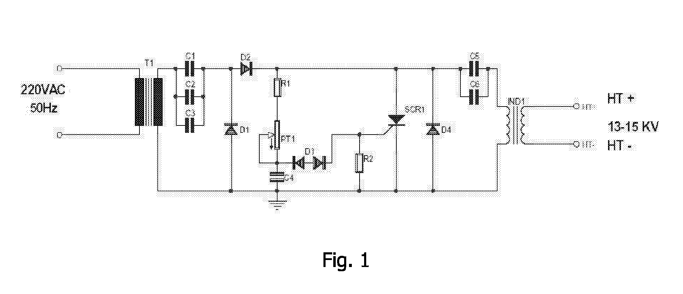

A circuit of an ozone generator according to the present invention is shown in fig. 1, wherein the symbols used have the following meanings: T= transformer; D = diode; R = resistor; C = capacitor; PT = potentiometer; SCR = controlled diode.

The ozone generator preferably also comprises a micro-controller card with an alphanumerical display and a relay for the activation of the generator and of the pump, and a PWM control for the regulation of the speed of the impellers.

Thus, another embodiment of the invention concerns an apparatus for sweetening drinkable water for domestic use, comprising ion exchange resins and an ozone generator.

More particularly, the apparatus according to the present invention comprises a vessel containing the ion exchange resins, a vessel containing the reservoir of salt for the regeneration of the resins, a valve for managing the regeneration phases and an ozone generator which is able to provide a flux of air or water containing from 0.010 to 1.0 mg/l of ozone for the time required to the disinfection.

A further embodiment of the invention concerns a method for the disinfection of an apparatus for sweetening of the domestic water comprising ion exchange resins characterized in that during one of the phases of regeneration of the resin, the resin is treated with a flux of water or air comprising ozone, such that the total quantity of ozone in relation with the volume of the resins is comprised between 0.20 and 20 mg/l, preferably between 0.5 and 10 mg/l.

It is possible to bubble air enriched with ozone directly in the resin containing vessel, with a frequency higher than the resins regeneration cycles.

The apparata according to the invention are apparata for domestic use, thus suitable to the installation in single apartment or in a condominium; they are characterized by a water flow rate comprised between 0.1 and 10 m³/h, preferably between 0.2 and 5 m³/h.

The apparatus according to the invention is basically very simple, being in all aspects similar to traditional apparata for sweetening domestic water by the use of ion exchange resins; it only differs in that it comprises an ozone generator as above described. The ozone generator generates an ozone containing air flux, which ozone has a concentration comprised between 0.010 and 1.0 mg/l and such flux is bubbled either directly in the vessel containing the ion exchange resins, or in the water feeded to the vessel, for example water used in one of the regeneration phases.

A traditional apparatus essentially consists of a vessel in which the ion exchange resins are located, a vessel wherein the salt for the regeneration of the resins is located, and a multifunction valve which allows to switch from the normal function of the apparatus to the various regeneration cycle.

A regeneration cycle normally consists of the following phases:
a) quick washing with water (about 5-10 min.)
b) equicurrent or countercurrent washing with brine (about 20-60 min.)
c) slow washing with water (about 20-60 min.)
d) quick washing with water (about 5-10 min.)

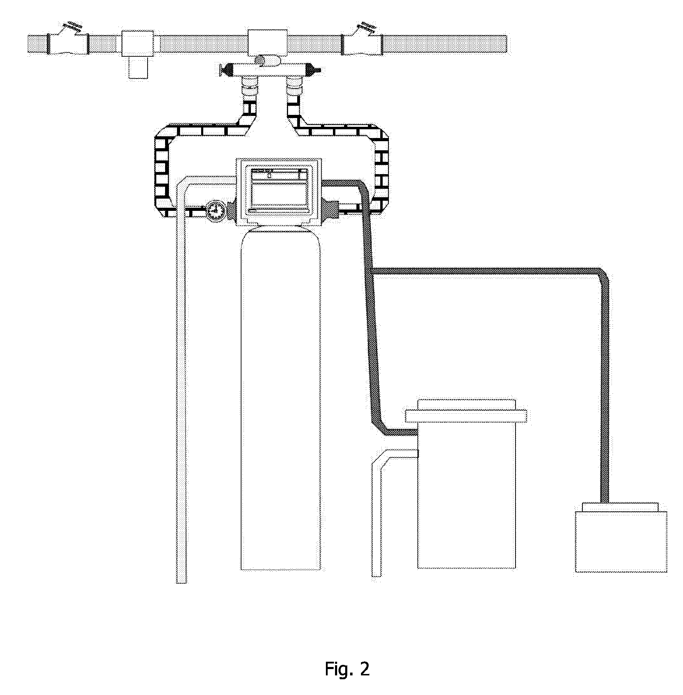

In traditional apparata chlorine is generated by a couple of electrodes directly immersed in the brine and is consequently introduced in the washing cycle with brine. When the multifunction valve activates the regeneration cycle, it also closes the circuit of the chlorine generator. The apparatus according to the present invention can instead introduce ozone during any of the washing cycles. Thus, it is possible to introduce ozone in the feeding flux of the brine to the resins (fig. 2). In this way it is possible to substitute a chlorine generator with an ozone generator without significantly changing the apparatus. It is enough to introduce a pipe tee to feed the ozone containing air to the brine before it enters the resins and to move the electric connection from the chlorine generator to the ozone generator. It is therefore possible to obtain an apparatus according to the present invention by substituting in a simple manner a chlorine generator with a dry ozone generator.

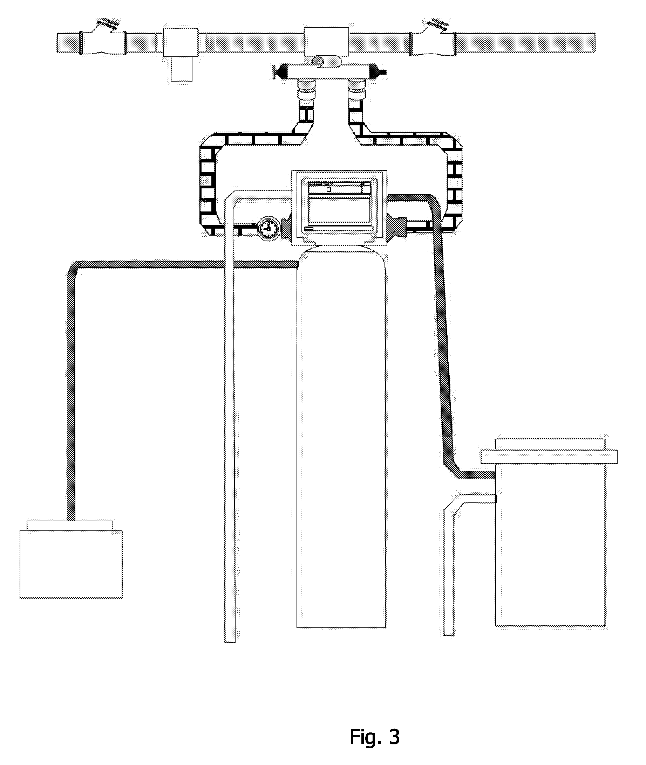

However, it is also possible to introduce ozone in any other phase of the regeneration cycle or to introduce a pause between a cycle and the following one to allow the disinfection with ozone. In this case it could be advantageous to introduce ozone in the resin containing vessel directly as a gas, i.e. by bubbling the ozone containing air produced by the ozone generator directly in the resin containing vessel (fig. 3). It is important to note that the ozone in water decompose quickly, in about ten minutes. Consequently, any washing phase is suitable for the introduction of ozone since there are needed only 10 min. between the end of the ozone introduction and the starting of the normal functioning of the resins. In fact, during this time, ozone decomposes and no relevant traces of it are left in the water leaving the apparatus. An advantage deriving from the direct introduction of ozone in the resin containing vessel is that it allows to perform disinfection cycles more frequently than regeneration cycles.

Experimental data show that small quantities of ozone suffices to kill bacterial flora formed in the normal functioning of the apparatus, but also to kill much higher concentrations deriving from contamination phenomena. The calculation of the time needed to complete disinfection can be made by taking into account that in order to achieve an optimal result, it is necessary to treat the resins with an amount of ozone comprised in the range from 0.20 to 20 mg/l (mg of ozone per litre of resin), preferably from 0.50 to 10 mg/l.

The ozone produced by the ozone generator can be used directly as a flux of air containing ozone or the flux of air containing ozone can be bubbled in water and the water enriched by the ozone can be feeded to the resin containing vessel.

The apparatus according to the present invention represents a guarantee of sterility of the treated waters even in very difficult working condition.

### Experimental part:

### Example 1. Disinfection of an ion exchange sweetener with ozone

The sweetener used is formed of a vessel having a volume of 10.5 I filled for about 2/3 with an ion exchange resin. The vessel was filled with about 5 I of water from the water net. The water was left in the vessel for time sufficiently long to guarantee that in the vessel the growth of micro-organisms was consistent (about 5 days).

In order to withdraw samples of water from the vessel, it was used a pump connected to a small pipe whose free end was introduced inside the vessel. This end was equipped with a filter to avoid that the resin was removed together with the water to be analyzed.

For the production of the ozone it was used a small cell for the generation of ozone (ozone generator) having nominal production capacity of 0.500 mg/l of ozone with a feeding of 150 NI/h of air at a constant pressure of 0.2 bar.

In this case, ozone was feeded to the ion exchange resin during the regeneration of the resins itself. The apparatus was designed in such a way that, during feeding of the regeneration brine of sodium chloride in water, together with the brine also the flux of air and ozone from the ozone generator was feeded.

In the specific case the regeneration/disinfection cycle was programmed as follow: feeding to the resins of the flux of air and ozone together with the brine water-sodium chloride; the duration of the feeding of the brine and ozone was 15 min.

Samples were taken before the treatment (1), at the end of the treatment (2) and 20 min. after the end of the treatment (3).

### Example 2 (comparative). Disinfection with chlorine of a sweetener based on ion exchange resins.

Example 1 was repeated using a chlorine generator instead of the ozone generator. Table 1 reports the amount of coliform bacteria measured in the two examples.

**Table 1**

| **Coliform bacteria (number/100 ml)** | | |
|---|---|---|
| Sample | Chlorine | Ozone |
| 1 | 6000 | 700 |
| 2 | 6000 | 100 |
| 3 | 5000 | 100 |

### Example 3 Bubbling of ozone in the resin containing vessel.

Scope of the example was an evaluation of the efficiency of a disinfection method of a ion exchange sweetener based on direct bubbling of air enriched with ozone in the resin containing vessel. More particularly, the efficiency of the treatment with ozone to eliminate possible bacterial colonies was determined by subjecting to microbiological analysis samples of water from the resins containing vessel. The samples were taken before and after disinfection. The disinfection was performed by bubbling a flux of air enriched with ozone for ten minutes. In the specific case, at the end of bubbling, there was a concentration of ozone in water of 0.040 mg/l.

Once bubbling was terminated, several samples were taken in time. The comparison between the concentration of bacterial colonies before and after disinfection allows the determination of the efficiency of disinfection with the system used. Furthermore, by taking several samples in time, it was possible to follow the trend of bacterial concentration in time. The analysis of this trend is useful to determine the time needed to completely destroy bacterial colonies and to evaluate the long term efficiency of the disinfection system used in the example.

The sweetener used is formed of a vessel having a volume of 10.5 I filled for about 2/3 with an ion exchange resin. The vessel was filled with about 5 I of water from the water net. The water was left in the vessel for a time sufficiently long to guarantee that in the vessel the growth of micro-organisms was consistent (about 7 days).

At the end of this time, 400 ml water samples were taken and subjected to microbiological analysis for the determination of the number of a enterococci, escherichia coli and coliform bacteria at 37°C. In the samples no escherichia coli was found and the amount of enterococci was extremely low. Samples were taken before treatment with ozone (sample 1), at the end of the 10 min. treatment (sample 2), after 5 min. (sample 3) and after 10 min. (sample 4).

The results of the microbiological analysis performed on the various samples of water are reported in table 2.

**Table 2**

| **Bubbling of ozone** | |
|---|---|
| Sample | Coliform bacteria n°/100 ml |
| 1 | 24.000 |
| 2 | 7.000 |
| 3 | 1.000 |
| 4 | 1.000 |

### Example 4. Treatment with ozone of water contaminated by escherichia coli.

The experimentation was conducted in order to verify the efficiency of ozone to kill bacterial colonies. The check of the efficiency of the treatment of escherichia coli containing water was conducted by subjecting to microbiological analysis samples of water taken at the entrance and at the exit of the water circuit prepared for the experimentation. The samples were taken at different times from the filling of a suitable vessel. Two consecutive tries were performed in order to determine the concentration of escherichia coli ad the entrance and the exit of the water circuit. In the two trials the air mass flow and the water volume flow rate were changed.

The water volume flow rate and air volume flow rate were measured. The air volume flow rate entered in the circuit was fixed at 45 I/h while the water volume flow rate circulating was equal to about 48 I/h. The amount of ozone dissolved, measured at the entrance of the vessel that closes the circuit (volume equal to about 2 I) was equal to about 0.020 mg/l.

Five water samples, each of 200 ml, were taken and subjected to a microbiological analysis for the determination of the concentration of escherichia coli colonies therein dissolved.

The five samples were taken according to the following scheme:

Sample 1: water contaminated by escherichia coli contains in the 10 I vessel, constituting the entrance of the experimental circuit.

Water contained in the 2 I vessel at the end of the experimental circuit, at the moment of the complete filling of the same (t=0')(sample 2); after 5 min. (sample 3); after 10 min. (sample 4) and after 15 min. (sample 5).

The results of the microbiological analysis performed on the five samples are reported in table 3.

**Tabl3. Escherichia coli concentration**

| Sample | (UFC⁽¹⁾/100 ml) |
|---|---|
| 1 | 5.900 |
| 2 | 5.700 |
| 3 | 5.400 |
| 4 | 3.700 |
| 5 | 400 |

| | |
|---|---|
| (1) UFC = Units forming Colonies | |

### Example 5. Treatment with ozone of water contaminated by escherichia coli.

Example 4 was repeated varying the air volume flow rate entering the circuit, which was increased to 135 l/h, while the water volume flow rate circulating was equal to 35 l/h. The amount of ozone dissolved, measured at the entrance of the vessel that closes the circuit was equal to about 0.025 mg/l. The analytical results are reported in table 4.

**Table 4. Escherichia coli concentration**

| Sample | UFC⁽¹⁾/100 ml |
|---|---|
| 1 | 5.600 |
| 2 | 4.500 |
| 3 | 4.600 |
| 4 | 3.700 |
| 5 | 250 |

| | |
|---|---|
| (1) UFC = Units forming Colonies | |

## Claims

1. Apparatus for sweetening drinkable water for domestic use, comprising ion exchange resins and an ozone generator.

2. Apparatus according to claim 1, comprising a vessel containing the ion exchange resins, a vessel containing the reservoir of salt for the regeneration of the resins, a valve for managing the regeneration phases and an ozone generator which is able to provide a flux of air or water containing from 0.010 to 1.0 mg/l of ozone for the time required to the disinfection.

3. Apparatus according to claims 1-2 wherein the total quantity of ozone added to the ion exchange resins during a regeneration is comprised between 0.20 and 20 mg/l in relation with the volume of the resins.

4. Apparatus according to claims 1-3 comprising an ozone generator comprising the following components: a double glass tube internally and externally coated with electrodes, to whom a tension of about 13-15 kV is applied, and a pump which generates a flux through the glass tube.

5. Ozone generator for use in an apparatus for the treatment of domestic water comprising the following components: a double glass tube internally and externally coated with electrodes, to whom a tension of about 13-15 kV is applied, and a pump which generates a flux through the glass tube, **characterized in that** the amount of ozone produced is comprised between 0.1 g/h and 5 g/h.

6. Method for the disinfection of an apparatus for sweetening of the domestic water comprising ion exchange resins **characterized in that** during one of the phases of the resins is treated with a flux of water or air comprising ozone, such that the total quantity of ozone in relation with the volume of the resins is comprised between 0.20 and 20 mg/l, preferably between 0.5 and 10 mg/l.

7. Method according to claim 6, wherein air enriched with ozone is bubbled directly in the resin containing vessel, with a frequency higher than the resins regeneration cycles.
